(19) **Europäisches Patentamt**

**European Patent Office**

**Office européen des brevets**

(11)    **EP 0 888 149 B1**

(12)    **EUROPEAN PATENT SPECIFICATION**

(45) Date of publication and mention
of the grant of the patent:
**27.07.2005   Bulletin 2005/30**

(51) Int Cl.⁷: **A61N 1/39**

(21) Application number: **96908727.9**

(86) International application number:
**PCT/US1996/003203**

(22) Date of filing: **01.03.1996**

(87) International publication number:
**WO 1997/031680 (04.09.1997 Gazette 1997/38)**

(54) **ELECTROTHERAPY APPARATUS**

ELEKTROTHERAPIEGERÄT

APPAREIL D'ELECTROTHERAPIE

(84) Designated Contracting States:
**DE GB**

(43) Date of publication of application:
**07.01.1999   Bulletin 1999/01**

(73) Proprietor: **Koninklijke Philips Electronics N.V.
5621 BA Eindhoven (NL)**

(72) Inventors:
 • **GLINER, Bradford, E.
  Bellevue, WA 98005 (US)**
 • **LYSTER, Thomas, D.
  Bothell, WA 98021 (US)**
 • **COLE, Clinton, S.
  Kirkland, WA 98033 (US)**

 • **POWERS, Daniel, J.
  Bainbridge Island, WA 98110 (US)**
 • **MORGAN, Carlton, B.
  Bainbridge Island, WA 98110 (US)**

(74) Representative: **van der Veer, Johannis Leendert
Philips
Intellectual Property & Standards
P.O. Box 220
5600 AE Eindhoven (NL)**

(56) References cited:
**EP-A- 0 437 104          EP-A- 0 457 604
WO-A-95/05215          WO-A-95/09673
WO-A-95/32020          US-A- 5 372 606**

## Description

This invention relates generally to an electrotherapy apparatus for delivering a shock to a patient's heart. In particular, this invention relates to an apparatus for using an external defibrillator to deliver a biphasic defibrillation shock to a patient's heart through electrodes attached to the patient.

Defibrillators apply pulses of electricity to a patient's heart to convert ventricular arrhythmias, such as ventricular fibrillation and ventricular taehycardia, to normal heart rhythms through the processes of defibrillation and cardioversion, respectively. There are two main classifications of defibrillators: external and implanted. Implantable defibrillators are surgically implanted in patients who have a high likelihood of needing electrotherapy in the future. Implanted defibrillators typically monitor the patient's heart activity and automatically supply electrotherapeutic pulses directly to the patient's heart when indicated. Thus, implanted defibrillators permit the patient to function in a somewhat normal fashion away from the watchful eye of medical personnel.

External defibrillators send electrical pulses to the patient's heart through electrodes applied to the patient's torso. External defibrillators are useful in the emergency room, the operating room, emergency medical vehicles or other situations where there may be an unanticipated need to provide electrotherapy to a patient on short notice. The advantage of external defibrillators is that they may be used on a patient as needed, then subsequently moved to be used with another patient. However, because external defibrillators deliver their electrotherapeutic pulses to the patient's heart indirectly (i.e., from the surface of the patient's skin rather than directly to the heart), they must operate at higher energies, voltages and/or currents than implanted defibrillators. The high energy, voltage and current requirements have made current external defibrillators large, heavy and expensive, particularly due to the large size of the capacitors or other energy storage media required by these prior art devices.

The time plot of the current or voltage pulse delivered by a defibrillator shows the defibrillator's characteristic waveform. Waveforms are characterized according to the shape, polarity, duration and number of pulse phases. Most current external defibrillators deliver monophasic current or voltage electrotherapeutic pulses, although some deliver biphasic sinusoidal pulses. Some prior art implantable defibrillators, on the other hand, use truncated exponential, biphasic waveforms. Examples of biphasic implantable defibrillators may be found in U.S. Patent No. 4,82 1,723 to Baker, Jr. et al.: U.S. Patent No. 5,083,562 to de Coriolis et al.; U.S. Patent No. 4,800,883 to Winstrom; U.S. Patent No. 4,850,357 to Bach, Jr.; U.S. Patent No. 4,953,551 to Mehra et al.; and U.S. Patent No. 5,372,606 to Lang et al.

Because each implanted defibrillator is dedicated to a single patient, its operating parameters, such as electrical pulse amplitudes and total energy delivered, may be effectively titrated to the physiology of the physiology of the patient to optimize the defibrillator's effectiveness. Thus, for example, the initial voltage, first phase duration and total pulse duration may be set when the device is implanted to deliver the desired amount of energy or to achieve that desired start and end voltage differential (i.e. a constant tilt). In contrast, because external defibrillator electrodes are not in direct contact with the patient's heart, and because external defibrillators must be able to be used on a variety of patients having a variety of physiological differences, external defibrillators must operate according to pulse amplitude and duration parameters that will be effective in most patients no matter what the patient's physiology. For example, the impedance presented by the tissue between external defibrillator electrodes and the patient's heart varies from patient to patient, thereby varying the intensity and waveform shape of the shock actually delivered to the patient's heart for a given initial pulse amplitude and duration. Pulse amplitudes and durations effective to treat low impedance patients do not necessarily deliver effective and energy efficient treatments to high impedance patients Ostroff(EP 0437 504 A4) developed a method of measuring the subthreshold defibrillation electrode resistance in order to provide a constant energy shock delivery. A known subthreshold current is sourced across the electrodes and the resulting voltage is measured. From the resulting voltage, the electrode resistance is calculated.

Prior art external defibrillators have not fully addressed the patient variability problem. One prior art approach to this problem was to provide the external defibrillator with multiple energy settings that could be selected by the user. A common protocol for using such a defibrillator was to attempt defibrillation at an initial setting suitable for defibrillating a patient of average impedance, then raise the energy setting for subsequent defibrillation attempts in the event that the initial setting failed. The repeated defibrillation attempts require additional energy and add to patient risk. What is needed, therefore, is an external defibrillation apparatus that maximizes energy efficiency (to minimize the size of the required energy storage medium) and maximizes therapeutic efficacy across an entire population of patients.

US-A-5372606 describes a method and an implanted defibrillating apparatus for delivering an adaptive n-phasic waveform to the heart in which the duration of the first phase is the lesser of (a) a predetermined duration and (b) the time taken for a predetermined tilt (as defined below).

WO-A-9505215 describes a method and apparatus for shaping the electrical waveform delivered by a defibrillator based on an electrical parameter measured during delivery of the waveform. In the case of high impedance patients, it is stated to be desirable to increase the duration of the first phase of the biphasic waveform relative to the duration of the second phase.

This invention provides an external defibrillator that automatically compensates for patient-to-patient impedance differences in the delivery of electrotherapeutic pulses for defibrillation and cardioversion. In a preferred embodiment, the defibrillator has an energy source that may be discharged through electrodes on the patient to provide a biphasic voltage or current pulse.

Thus, in accordance with the present invention there is provided an apparatus for applying electrotherapy to a patient as defined in claim 1.

Figure 1 is a schematic representation of a low-tilt biphasic electrotherapeutic waveform according to a first arrangement, which is not an embodiment of the present invention.

Figure 2 is a schematic representation of a high-tilt biphasic electrotherapeutic waveform according to the first arrangement.

Figure 3 is a flow chart demonstrating part of an electrotherapy method according to a second arrangement constituting a preferred embodiment of the present invention.

Figure 4 is a schematic representation of a biphasic waveform delivered according to the second arrangement.

Figure 5 is a schematic representation of a biphasic waveform delivered according to the second arrangement.

Figure 6 is a block diagram of a defibrillator system according to a preferred embodiment of this invention.

Figure 7 is a schematic circuit-diagram of a defibrillator system according to a preferred embodiment of this invention.

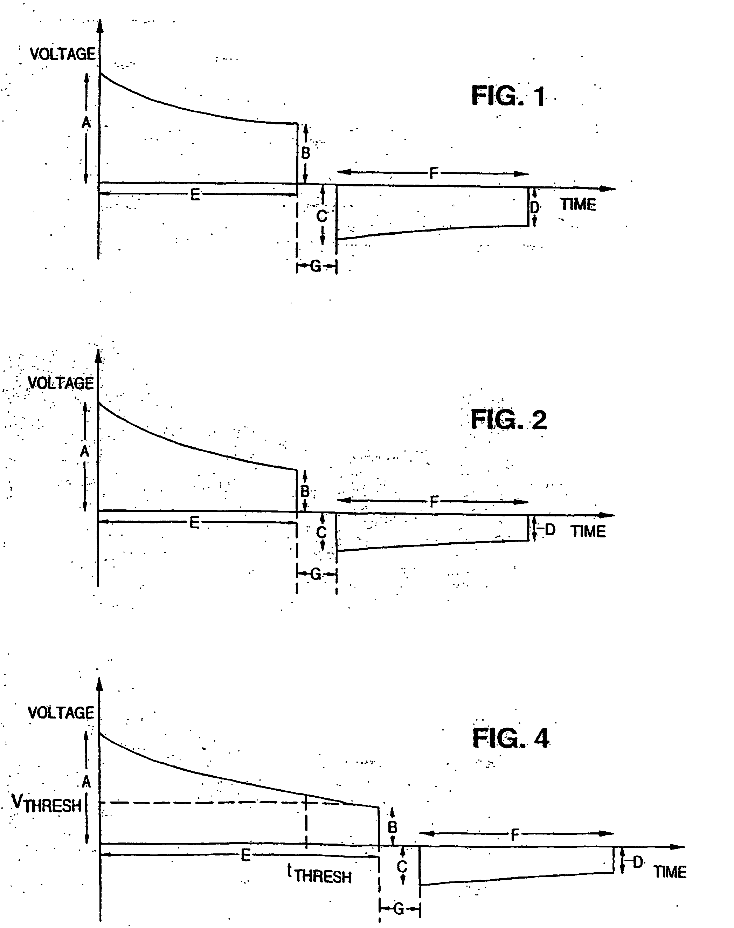

Figures 1 and 2, which does not relate to an embodiment of the present invention, illustrate the patient-to-patient differences that an external defibrillator design must take into account. These figures are schematic representations of truncated exponential biphasic waveforms delivered to two different patients from an external defibrillator for defibrillation or cardioversion. In these drawings, the vertical axis is voltage, and the horizontal axis is time. The principles discussed here are applicable to waveforms described in terms of current versus time as well, however.

The waveform shown in Figure 1 is called a low-tilt waveform, and the waveform shown in Figure 2 is called a high-tilt waveform, where tilt H is defined as a percent as follows:

$$H = \frac{|A|-|D|}{|A|} \ x \ 100$$

As shown in Figures 1 and 2, A is the initial first phase voltage and D is the second phase terminal voltage. The first phase terminal voltage B results from the exponential decay over time of the initial voltage A through the patient, and the second phase terminal voltage D results from the exponential decay of the second phase initial voltage C in the same manner. The starting voltages and first and second phase durations of the Figure 1 and Figure 2 waveforms are the same; the differences in end voltages B and D reflect differences in patient impedance.

Prior art disclosures of the use of truncated exponential biphasic waveforms in implantable defibrillators have provided little guidance for the design of an external defibrillator that will achieve acceptable defibrillation or cardioversion rates across a wide population of patients. The defibrillator operating voltages and energy delivery requirements affect the size, cost, weight and availability of components. In particular, operating voltage requirements affect the choice of switch and capacitor technologies. Total energy delivery requirements affect defibrillator battery and capacitor choices.

We have determined that, for a given patient, externally-applied truncated exponential biphasic waveforms defibrillate at lower voltages and at lower total delivered energies than externally-applied monophasic waveforms. In addition, we have determined that there is a complex relationship between total pulse duration, first to second phase duration ratio, initial voltage, total energy and total tilt.

Up to a point, the more energy delivered to a patient in an electrotherapeutic pulse, the more likely the defibrillation attempt will succeed. Low-tilt biphasic waveforms achieve effective defibrillation rates with less delivered energy than high-tilt waveforms. However, low-tilt waveforms are energy inefficient, since much of the stored energy is not delivered to the patient. On the other hand, defibrillators delivering high-tilt biphasic waveforms deliver more of the stored energy to the patient than defibrillators delivering low-tilt waveforms while maintaining high efficacy up to a certain critical tilt value. Thus, for a given capacitor, a given initial voltage and fixed phase durations, high impedance patients receive a waveform with less total energy and lower peak currents but better conversion properties per unit of energy delivered, and low impedance patients receive a waveform with more delivered energy and higher peak currents. There appears to be an optimum tilt range in which high and low impedance patients will receive effective and efficient therapy. An optimum capacitor charged to a predetermined voltage can be chosen to deliver an effective and efficient waveform across a population of patients having a variety of physiological differences.

The preset parameters of the waveforms shown in Figure 1 and 2 are the initial voltage A of the first phase of the pulse, the duration E of the first phase, the interphase duration G, and the duration F of the second phase. The terminal voltage B of the first phase, the initial voltage C of the second phase, and the terminal voltage D of the second phase are dependent upon the physiological parameters of the patient and the physical connection between the electrodes and the patient.

For example, if the patient impedance (i.e., the total impedance between the two electrodes) is high, the amount of voltage drop (exponential decay) from the initial voltage A to the terminal voltage B during time E will be lower (Figure 1) than if the patient impedance is low (Figure 2). The same is true for the initial and terminal voltages of the second phase during time F. The values of A, E, G and F are set to optimize defibrillation and/or cardioversion efficacy across a population of patients.

Thus, high impedance patients receive a low-tilt waveform that is more effective per unit of delivered energy, and low impedance patients receive a high-tilt waveform that delivers more of the stored energy and is therefore more energy efficient.

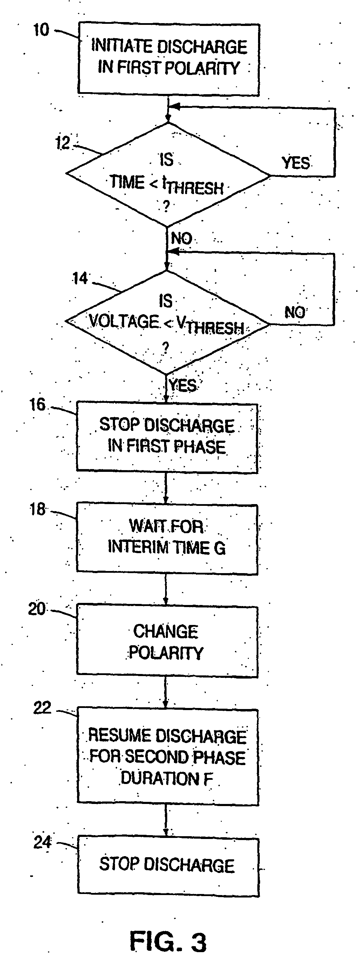

Another feature of biphasic waveforms is that waveforms with relatively longer first phases have better conversion properties than waveforms with equal or shorter first phases, provided the total duration exceeds a critical minimum. Therefore, in the case of high impedance patients, it may be desirable to extend the fist phase of the biphasic waveform (while the second phase duration is kept constant) to increase the overall efficacy of the electrotherapy by delivering a more efficacious waveform and to increase the total amount of energy delivered. Figures 3-5 demonstrate a defibrillation arrangement according to a preferred embodiment of the invention in which information related to patient impedance is fed back to the defibrillator to change the parameters of the delivered electrotherapeutic pulse.

Figure 3 is a flow chart showing the method steps following the decision (by an operator or by the defibrillator itself) to apply an electrotherapeutic shock to the patient through electrodes attached to the patient and charging of the energy source, e.g., the defibrillator's capacitor or capacitor bank, to the initial first phase voltage A. Block 10 represents initiation of the first phase of the pulse in a first polarity. Discharge may be initiated manually by the user or automatically in response to patient heart activity measurements (e.g., ECG signals) received by the defibrillator through the electrodes and analyzed by the defibrillator controller in a manner known in the art.

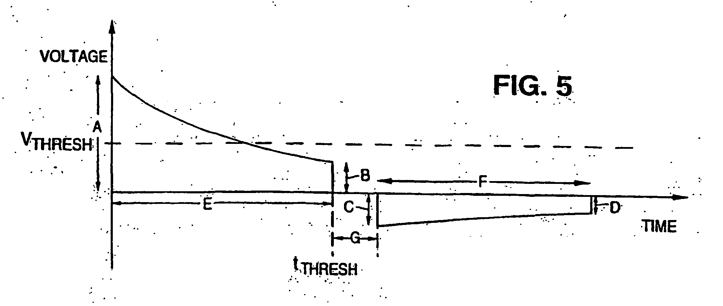

Discharge of the first phase continues for at least a threshold time $t_{THRESH}$, as shown by block 12 of Figure 3. If, at the end of time $t_{THRESH}$, the voltage measured across the energy source has not dropped below the minimum first phase terminal voltage threshold $V_{THRESH}$, first phase discharge continues, as shown in block 14 of Figure 3. For high impedance patients, this situation results in an extension of the first phase duration beyond $t_{THRESH}$, as shown in Figure 4, until the measured voltage drops below the threshold $V_{THRESH}$. Discharge then ends to complete the first phase, as represented by block 16 of Figure 3. If, on the other hand, the patient has low impedance, the voltage will have dropped below $V_{THRESH}$ when the time threshold is reached, resulting in a waveform like the one shown in Figure 5.

At the end of the first phase, and after a predetermined interim period G, the polarity of the energy source connection to the electrodes is switched, as represented by blocks 18 and 20 of Figure 3. Discharge of the second phase of the biphasic pulse then commences and continues for a predetermined second phase duration F, as represented by block 26 of Figure 3, then ceases. This compensating electrotherapy method ensures that the energy is delivered by the defibrillator in the most efficacious manner by providing for a minimum waveform tilt and by extending the first phase duration to meet the requirements of a particular patient.

Because this method increases the waveform tilt for high impedance patients and delivers more of the energy from the energy source than a method without compensation, the defibrillator's energy source can be smaller than in prior art external defibrillators, thereby minimizing defibrillator size, weight and expense. It should be noted that the waveforms shown in Figures 4 and 5 could be expressed in terms of current versus time using a predetermined current threshold value without departing from the scope of the invention.

In alternative embodiments of this invention, the second phase pulse could be a function of the first phase voltage, current or time instead of having a fixed time duration. In addition, any of the above embodiments could provide for alternating initial polarities in successive monophasic or biphasic pulses. In other words, if in the first biphasic waveform delivered by the system the first phase is a positive voltage or current pulse followed by a second phase negative voltage or current pulse, the second biphasic waveform delivered by the system would be a negative first phase voltage or current pulse followed by a positive second phase voltage or current pulse. This arrangement would minimize electrode, polarization, i.e., build-up of charge on the electrodes.

For the above defibrillator method, the initial first phase voltage A may be the same for all patients or it may be selected automatically or by the defibrillator user. For example, the defibrillator may have a selection of initial voltage settings, one for an infant, a second for an adult, and a third for use in open heart surgery.

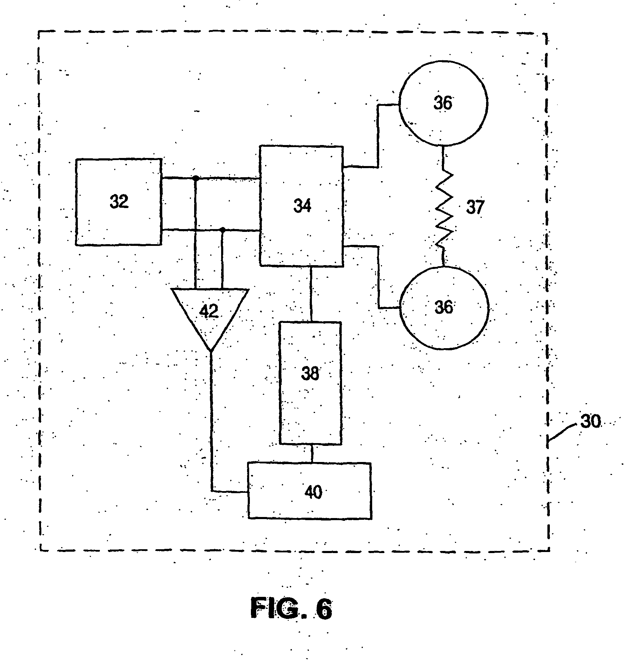

Figure 6 is a schematic block diagram of a defibrillator system according to a preferred embodiment of this invention. The defibrillator system 30 comprises an energy source 32 to provide the voltage or current pulses described above. In one preferred embodiment, energy source 32 is a single capacitor or a capacitor bank arranged to act as a single capacitor. A connecting mechanism 34 selectively connects and disconnects energy source 32 to and from a pair of electrodes 36 electrically attached to a patient, represented here as a resistive load 37. The connections between the electrodes and the energy source may be in either of two polarities with respect to positive and negative terminals on the energy source.

The defibrillator system is controlled by a controller 38. Specifically, controller 38 operates the connecting mechanism 34 to connect energy source 32 with electrodes 36 in one of the two polarities or to disconnect energy source 32 from electrodes 36. Controller 38 receives timing information from a timer 40, and timer 40 receives electrical information from electrical sensor 42 connected across energy source 32. In some preferred embodiments, sensor 42 is a voltage sensor; in other preferred embodiments, sensor 42 is a current sensor.

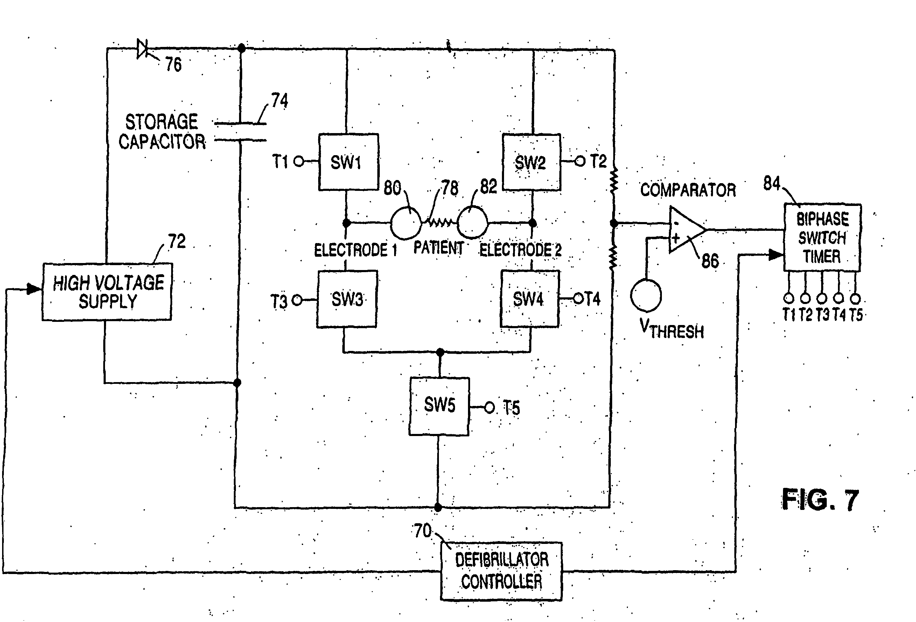

Figure 7 is a schematic circuit diagram illustrating a device according to the preferred embodiment discussed above. Defibrillator controller 70 activates a high voltage power supply 72 to charge storage capacitor 74 via diode 76 to a predetermined voltage. During this period, switches SW1, SW2, SW3 and SW4 are turned-off so that no voltage is applied to the patient (represented here as resistor 78) connected between electrodes 80 and 82. SW5 is turned on during this time.

After charging the capacitor, controller 70 deactivates supply 72 and activates biphase switch timer 84. Timer 84 initiates discharge of the first phase of the biphasic waveform through the patient in a first polarity by simultaneously turning on switches SW1 and SW4 via control signals T1 and T4, while switch SW5 remains on to deliver the initial voltage A through electrodes 80 and 82 to the patient 78.

Depending on the operating mode, delivery of the first phase of the biphasic pulse may be terminated by the timer 84 after the end of a predetermined period or when the voltage across the electrodes has dropped below a predetermined value as measured by comparator 86. Timer 84 terminates pulse delivery by turning off switch SW5 via control signal T5, followed by turning off switches SW1 and SW4. The voltage across electrodes 80 and 82 then returns to zero. During the interim period G, SW5 is turned on to prepare for the second phase. After the end of interim period G, timer 84 initiates delivery of the second phase by simultaneously turning on switches SW2 and SW3 via control signals T2 and T3 while switch SW5 remains on. This configuration applies voltage from the capacitor to the electrodes at an initial second phase voltage C and in a polarity opposite to the first polarity. Timer 84 terminates delivery of the second phase by turning off switch SW5 via control signal T5, followed by turning off switches SW2 and SW3. The second phase may be terminated at the end of a predetermined period or when the voltage measured by comparator 86 drops below a second phase termination voltage threshold.

In a preferred embodiment, switch SW5 is an insulated gate bipolar transistor (IGBT) and switches SW1-SW4 are silicon-controlled rectifiers (SCRs). The SCRs are avalanche-type switches which can be turned on to a conductive state by me application of a control signal, but cannot be turned off until the current through the switch falls to zero or near zero. Thus, the five switches can be configured so that any of the switches SW1-SW4 will close when SW5 is closed and will reopen only upon application of a specific control signal to SW5.

This design has the further advantage that switch SW5 does not need to withstand the maximum capacitor voltage. The maximum voltage that will be applied across switch SW5 will occur when the first phase is terminated by turning SW5 off, at which time the capacitor voltage has decayed to some fraction of its initial value.

Other switches and switch configurations may be used, of course without departing from the scope of the invention. In addition, the defibrillator configurations of Figures 10 and 11 may be used to deliver electric pulses of any polarity, amplitude, and duration singly and in any combination.

While the invention has been discussed with reference to external defibrillators, one or more aspects of the invention would be applicable to implantable defibrillators as well. Other modifications will be apparent to those skilled in the art.

**Claims**

1. An electrotherapy apparatus for delivering a shock to a patient's heart comprising:

> an energy source (32);
> a connecting mechanism (34) coupled to said energy source;
> a pair of electrodes (36) for coupling to said patient and further coupled to said connecting mechanism;
> an electrical sensor (42) coupled to said energy source (32) so as, in use, to measure an electrical unit associated with the patient;
> a timer (40) coupled to said electrical sensor (42); and
> a controller (38) coupled to said connecting mechanism (34) and to said timer (40) and arranged to discharge said energy source (32) across said electrodes (36) in a first polarity;

> **characterised in that** said controller (38) is arranged to control the duration of the discharge to be always the greater of (a) a first single predetermined time period and (b) the time taken for the measured electrical unit to decay to a predetermined terminal level.

2. An apparatus as claimed in claim 1, wherein said electrical unit comprises voltage.

3. An apparatus as claimed in claim 1, wherein said electrical unit comprises current.

4. An apparatus as claimed in any preceding claim, wherein said controller (38) is arranged to discharge said energy source (32) across said electrodes (36) in a second polarity.

5. An apparatus as claimed in claim 4, wherein said controller (38) is arranged to discharge said energy source (32) across said electrodes (36) in a second polarity for a second predetermined time period.

6. An apparatus as claimed in claim 4, wherein said controller (38) is arranged to discharge said energy source (32) across said electrodes (36) in a second polarity until an electrical unit measured across said electrodes (36) decays to a second predetermined terminal level.

**Patentansprüche**

1. Elektrotherapiegerät zur Abgabe eines Schocks an das Herz eines Patienten, wobei das Elektrotherapiegerät Folgendes umfasst:

   eine Energiequelle (32);
   einen Verbindungsmechanismus (34), der mit der genannten Energiequelle gekoppelt ist;
   ein Elektrodenpaar (36) zur Kopplung des genannten Patienten und das weiterhin mit dem genannten Verbindungsmechanismus gekoppelt ist;
   einen elektrischen Sensor (42), der mit der genannten Energiequelle (32) gekoppelt ist, um im Betrieb eine zu dem Patienten gehörende elektrische Einheit zu messen;
   einen Timer (40), der mit dem genannten elektrischen Sensor (42) gekoppelt ist; und
   eine Steuereinheit (38), die mit dem genannten Verbindungsmechanismus (34) und dem genannten Timer (40) gekoppelt ist und vorgesehen ist, um die genannte Energiequelle (32) über die genannten Elektroden (36) mit einer ersten Polarität zu entladen;

   **dadurch gekennzeichnet, dass** die genannte Steuereinheit (38) vorgesehen ist, um die Dauer der Entladung so zu steuern, dass sie immer entweder (a) einer ersten einzelnen vorgegebenen Zeitdauer oder (b) der Zeit entspricht, die die gemessene elektrische Einheit benötigt, um auf ein vorgegebenes Abschlussniveau abzuklingen, je nachdem, welcher Wert größer ist.

2. Gerät nach Anspruch 1, wobei die genannte elektrische Einheit die Spannung umfasst.

3. Gerät nach Anspruch 1, wobei die genannte elektrische Einheit den Strom umfasst.

4. Gerät nach einem der vorhergehenden Ansprüche, wobei die genannte Steuereinheit (38) vorgesehen ist, um die genannte Energiequelle (32) über die genannten Elektroden (36) mit einer zweiten Polarität zu entladen.

5. Gerät nach Anspruch 4, wobei die genannte Steuereinheit (38) vorgesehen ist, um die genannte Energiequelle (32) über die genannten Elektroden (36) mit einer zweiten Polarität für eine zweite vorgegebene Zeitdauer zu entladen.

6. Gerät nach Anspruch 4, wobei die genannte Steuereinheit (38) vorgesehen ist, um die genannte Energiequelle (32) über die genannten Elektroden (36) mit einer zweiten Polarität zu entladen, bis eine an den genannten Elektroden (36) gemessene elektrische Einheit auf ein zweites vorgegebenes Abschlussniveau abgeklungen ist.

**Revendications**

1. Appareil d'éléctrothérapie pour donner un choc au coeur d'un patient, comprenant:

   - une source d'énergie (32);
   - un mécanisme de connexion (34) couplé à ladite source d'énergie;
   - une paire d'électrodes (36) pour couplage audit patient et en outre couplée audit mécanisme de connexion;
   - un capteur électrique (42) couplé à ladite source d'énergie (32) pour, en fonctionnement, mesurer une unité électrique associée au patient;
   - un temporisateur (40) couplé audit capteur électrique (42);
   - un dispositif de commande (38) couplé audit mécanisme de connexion (34) et audit temporisateur (40) et agencé pour décharger ladite source d'énergie (32) à travers lesdites électrodes (36) dans une première polarité;

   **caractérisé en ce que** ledit dispositif de commande (38) est agencé pour commander la durée de la décharge pour qu'elle soit toujours la plus grande (a) d'une seule première période de temps prédéterminée et (b) le temps pris par l'unité électrique mesurée pour diminuer à un niveau final prédéterminé.

2. Appareil selon la revendication 1, **caractérisé en ce que** ladite unité électrique comprend une tension.

3. Appareil selon la revendication 1, **caractérisé en ce que** ladite unité électrique comprend un courant.

**4.** Appareil selon l'une quelconque des revendications précédentes, **caractérisé en ce que** ledit dispositif de commande (38) est agencé pour décharger ladite source d'énergie (32) à travers lesdites électrodes (36) dans une deuxième polarité.

**5.** Appareil selon la revendication 4, **caractérisé en ce que** ledit dispositif de commande (38) est agencé pour décharger ladite source d'énergie (32) à travers lesdites électrodes (36) dans une deuxième polarité pendant une deuxième période de temps prédéterminée.

**6.** Appareil selon la revendication 4, **caractérisé en ce que** ledit dispositif de commande (38) est agencé pour décharger ladite source d'énergie (32) à travers lesdites électrodes (36) dans une deuxième polarité jusqu'à ce qu'une unité électrique mesurée aux bornes desdites électrodes (36) diminue à un deuxième niveau final prédéterminé.

FIG. 1

FIG. 2

FIG. 4

**FIG. 3**

Flowchart:

10 — INITIATE DISCHARGE IN FIRST POLARITY

12 — IS TIME < $t_{THRESH}$ ? → YES (loop back)

NO ↓

14 — IS VOLTAGE < $V_{THRESH}$ ? → NO (loop back)

YES ↓

16 — STOP DISCHARGE IN FIRST PHASE

18 — WAIT FOR INTERIM TIME G

20 — CHANGE POLARITY

22 — RESUME DISCHARGE FOR SECOND PHASE DURATION F

24 — STOP DISCHARGE

FIG. 5

**FIG. 6**

FIG. 7

EP 0 888 149 B1